# EUROPEAN PATENT APPLICATION

(11) **EP 1 647 552 A1**
(43) Date of publication of application: **19.04.2006**
(21) Application number: 04425752.5
(22) Date of filing: 08.10.2004
(51) Int. Cl.: C07D 305/14

(54) **Semisynthesis process for the preparation of 10-deacetyl-n-debenzoyl-paclitaxel**

(71) Applicant: INDENA S.p.A., 20139 Milano (IT)
(72) Inventor: Gabetta, Bruno, 20139 Milano (IT); Gambini, Andrea, 20139 Milano (IT); Bombardelli, Ezio, 20141 Milano (IT); Fontana, Gabriele, 20139 Milano (IT)
(74) Representative: Minoja, Fabrizio

(57) **Abstract**

The invention relates to a process for the preparation of 10-deacetyl-N-debenzoyl-paclitaxel (**I**) 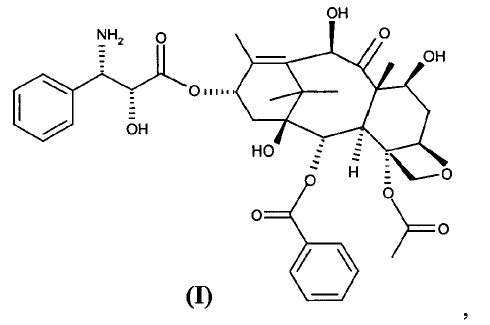 a synthon useful for the preparation of taxanes with antitumour activity, and intermediates for the preparation thereof.

## Description

### Field of the invention

Object of the present invention is a new semisynthesis process for the preparation of 10-deacetyl-N-debenzoyl-paclitaxel **(I)**, a useful synthon for the preparation of taxanes with anti-tumour activity.

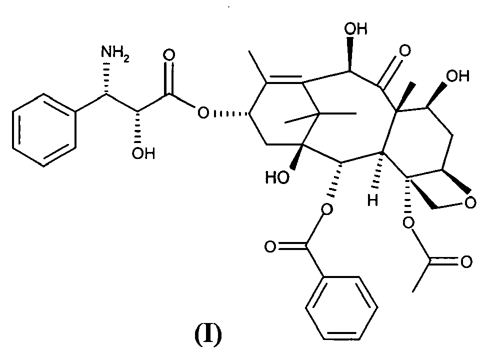

### State of the art

A process comprising the esterification with oxazolidines of formula **(II)** 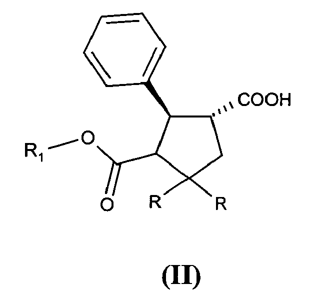 of 10-deacetylbaccatin protected at the 7- and 10-positions of formula **(III)** 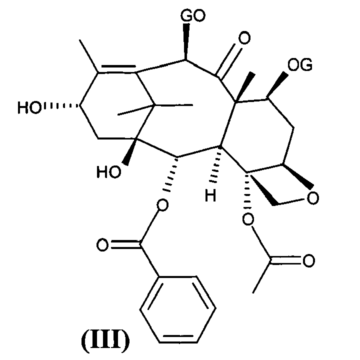 to give esters of formula **(IV)** 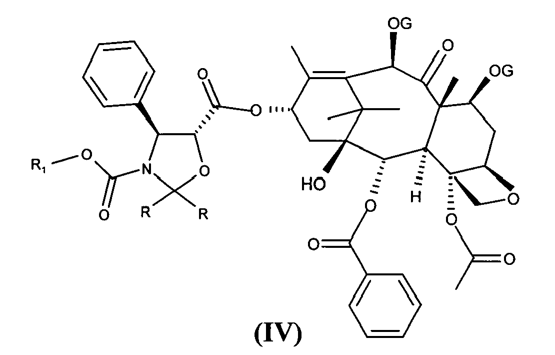 has been disclosed in WO 94/07877 for the synthesis of synthon **(I)**, reported in the literature in the early '90s (F. Gueritte-Voegelein et al., J. Med. Chem. 34, 992, 1991).

Liberation of the amino function at the 3'-position and hydroxy groups at the 2'-,7- and 10-positions from the esters of formula **(IV)** affords synthon **(I)**.

In particular, according to the above-cited patent application, groups R can be hydrogen, alkyl, alkoxy or variously-substituted phenyl and R₁ is alkyl substituted with one or more chlorine atoms. Groups G are alkylsilyl or R₁-O-CO- groups wherein R₁ is as defined above.

Starting from the intermediates of formula **(IV)**, the hydroxy and the amino functions are liberated by reduction with zinc and acids and, when groups G are alkylsilyl, the hydroxy functions are liberated by acid treatment, for example with hydrofluoric acid.

### Description of the invention

The present invention relates to a process for the synthesis of synthon **(I)** 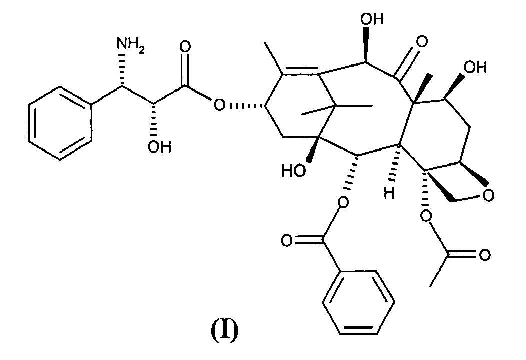 in high yield and quality, without the need for polluting or difficult to handle reagents, such as zinc and hydrofluoric acid.

The process consists in the reaction of 2-(2,4-dimethoxyphenyl)-3-(2-nitrobenzenesulfenyl)-4(*S*)-phenyl-5(*R*)-oxazolidinecarboxylic acid **(V)** 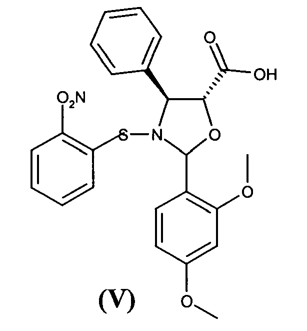 with 10-deacetyl-bis-7,10-trichloroacetylbaccatin III **(VI)** 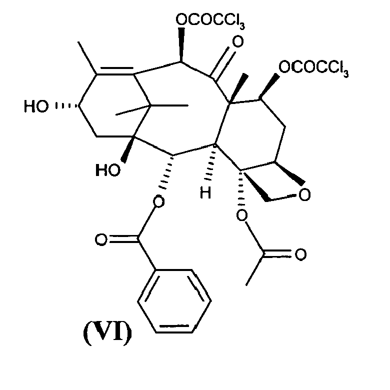 to give the ester **(VII)** 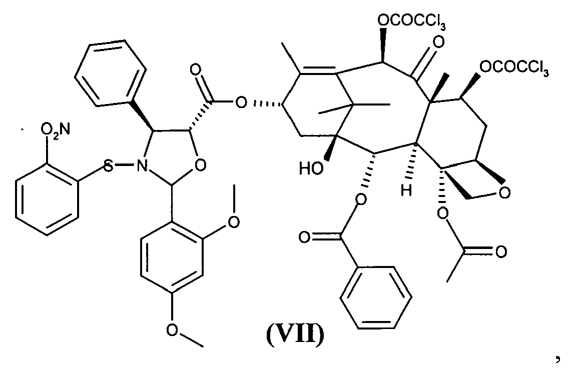 wherefrom synthon **(I)** is obtained after liberation of the amino and hydroxy functions.

The compound of formula **(VII)** is novel and is a further object of the present invention.

The oxazolidine acid **(V)** consists of a mixture of the 2R and 2S epimers, equally useful in the synthesis, since the chiral center at the 2-position of the oxazoline ring is removed from intermediate **(VII)** upon liberation of the hydroxy and amino functions. In other words, the relative ratio between the diastereoisomers does not impair the performance of the synthesis.

The oxazolidine acid **(V)** is easily prepared by acid treatment of the corresponding alkali salts, whose preparation has been disclosed in WO 03/087077 A1.

Compared to other oxazolidine acids, acid **(V)** is characterised by remarkable stability; which allows to easily carry out the esterification with synthon **(VI)**.

Moreover, after the esterification, the liberation of the amino and hydroxy functions contained in the acid residue can be easily carried out by treatment with acids, without the need to adopt drastic conditions.

The taxane synthon **(VI)** can be obtained from the natural metabolite 10-deacetylbaccatin III through esterification of the 7- and 10-positions by treatment with trichloroacetic acid activated derivatives, according to known esterification methods. Preferably, synthon **(VI)** is obtained by reaction with trichloroacetic acid chloride at a temperature around 0°C, using pyridine as the solvent.

According to the present invention, the esterification of **(VI)** with the oxazolidine acid **(V)** to give **(VII)** can be carried out in the presence of a condensing agent, such as a diimide, for example dicyclohexylcarbodiimide, and an activating agent, for example 4-dimethylamino-pyridine or 4-pyrrolidino-pyridine in a solvent selected from an ether, such as ethyl ether, diisopropyl ether, tetrahydrofurane or dioxane; an ester, such as ethyl, propyl or butyl acetate; an aromatic hydrocarbon, such as benzene, toluene or *o*-, *m-, p*-xylene; or a halogenated aliphatic hydrocarbon, for example methylene chloride, chloroform or dichloroethane. Carrying out the esterification in methylene chloride at the temperature of about 20°C is particularly advantageous.

The preparation of synthon **(I)** from ester **(VII)** requires removal of the trichloroacetyl groups from the 7- and 10-positions and liberation of the amino and hydroxy functions from the oxazolidine residue.

As mentioned above, the amino and hydroxy functions can be easily liberated from the oxazolidine residue by acid treatment. On the contrary, the hydrolysis of the trichloroacetic esters can be conveniently carried out by mild alkaline treatment, preferably by reaction with ammonium hydroxide.

It has been observed that, if the liberation of the amino and hydroxy functions from the oxazolidine residue is carried out first, massive migration of a trichloroacetyl group from the baccatin residue to the free amino function occurs, with consequent formation of a trichloroacetamido function, which could be transformed in an amino function only under conditions that would be detrimental to the structure of the baccatin skeleton. As a consequence, the preparation of synthon **(I)** requires first the removal of the trichloroacetic groups at the 7- and 10-positions of **(VII)** to give ester **(VIII).**

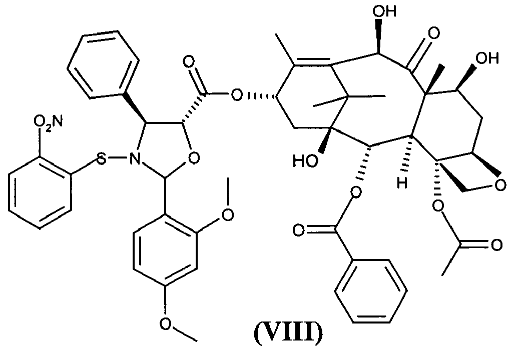

Also the compound of formula **(VIII)** is novel and is a further object of the present invention. Preferably, the removal of the trichloroacetic groups is carried out at room temperature by treatment with ammonium hydroxide in tetrahydrofuran as the solvent.

The liberation of the amino and hydroxy functions is carried out by treatment with acids, preferably with aqueous hydrochloric acid, in alcoholic solution, for example in methanol at a temperature of about 20°C. After dilution with water and removal of reaction by-products with organic solvents, such as aliphatic hydrocarbons and halogenated haliphatic hydrocarbons, for example *n*-hexane and methylene chloride, synthon **(I)** is isolated by alkalinization of the aqueous phase, extraction in an organic solvent, for example methylene chloride or ethyl acetate, concentration and precipitation in an aliphatic hydrocarbon, such as *n*-hexane. The process of the invention provides synthon **(I)** with purity higher than 98%, without chromatographic purifications.

The invention will be now illustrated in more detail in the following examples.

### EXAMPLES

### Example 1 - 10-Deacetyl-7,10-bistrichloroacetylbaccatin III (VI)

10-Deacetylbaccatin III (15 g) is treated with 6.6 ml of trichloroacetyl chloride in 60 ml of pyridine at 0-5°C for 1 hour under stirring. The mixture is diluted with 100 ml of methylene chloride and 100 ml 4 of N hydrochloric acid. The phases are separated and the organic one is washed with 100 of ml 4 N hydrochloric acid and 50 ml of water saturated with sodium chloride. The organic phase is concentrated under vacuum and the residue is taken up with 100 ml of toluene. Product **(VI)** is collected by filtration and dried under vacuum at 50°C.

Yield: 25 g.

### Example 2 - 2-(2,4-Dimethoxyphenyl)-3-(2-nitrobenzensulfenyl)-4(S)-phenyl-5(R)-oxazolidine carboxylic acid, 10-deacetyl-7,10-bis-trichloroacetylbaccatin III 13-yl-ester (VII)

A solution containing 10.3 g of (V) in the form of sodium salt in 100 ml of water is cooled to 0-5°C and adjusted to pH 2-3 with a 2 M sodium bisulfate solution. Acid **(V)** is extracted with 120 ml of methylene chloride and the organic phase, after drying over magnesium sulfate, is treated with 12 g of **(VI)**, 0.17 g of 4-dimethylaminopyridine and 5.88 of dicyclohexylcarbodimide for 1 hour at room temperature. The formed dicyclohexylurea is filtered off and the chloromethylene solution is evaporated to dryness yielding 24 g of **(VII)**.

### Example 3 - 2-(2,4-Dimethoxyphenyl)-3-(2-nitrobenzensulfenyl)-4(S)-phenyl-5(R) -oxazolidine carboxylic acid, 10-deacetylbaccatin III 13-yl-ester (VIII)

A solution containing 24 g of **(VII)** in 100 ml of tetrahydrofuran is treated for two hours at room temperature with 1.8 ml of 33% ammonium hydroxide. The solution is concentrated under vacuum and diluted with 125 ml of methanol. After precipitation, product **(VIII)** is collected by filtration and vacuum dried at 50°C. Yield: 13 g.

### Example 4 - 10-Deacetyl-N-debenzoyl-paclitaxel (I)

A suspension of 13 g of **(VIII)** in 260 ml of methanol is treated for 30 minutes at room temperature under stirring with 4.2 ml of concentrated aqueous hydrochloric acid diluted with 130 ml methanol. The reaction mixture is extracted twice with 300 ml of hexane and once with 150 ml of methylene chloride. The organic phases are discarded and the hydro-alcoholic phase is treated with 3 ml of concentrated ammonium hydroxide. After 5 extractions with 100 ml of methylene chloride, the organic phases are pooled, dried over sodium sulphate, filtered and concentrated to small volume. 8.0 g of **(I)** precipitates by dilution with *n*-hexane and is vacuum dried at 40°C. The obtained product has a purity higher than 98% (HPLC analysis).

## Claims

1. A process for the preparation of 10-deacetyl-N-debenzoyl-paclitaxel **(I)** 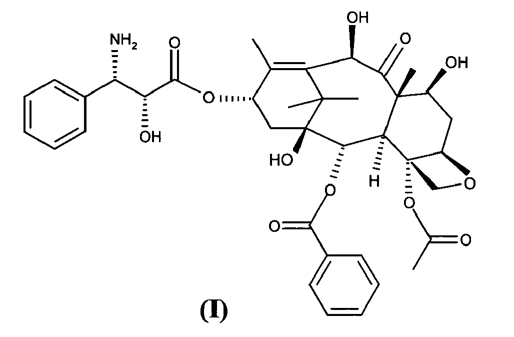 comprising the following steps:
a) reaction of 2-(2,4-dimethoxyphenyl)-3-(2-nitrobenzenesulfenyl)-4(*S*)-phenyl-5(*R*)-oxazolidinecarboxylic acid **(V)** 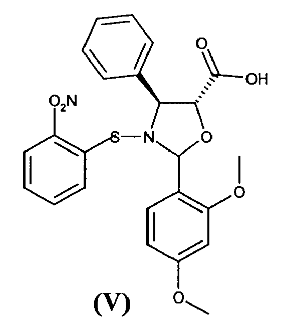 with 10-deacetyl-bis-7,10-trichloroacetylbaccatin III **(VI)** 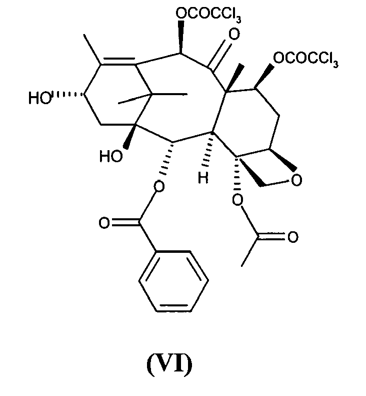 to give 2-(2,4-dimethoxyphenyl)-3-(2-nitrobenzensulfenyl)-4(*S*)-phenyl-5(*R*)-oxazolidine carboxylic acid, 10-deacetyl-7,10-bis-trichloroacetylbaccatin III 13-yl-ester **(VII)** 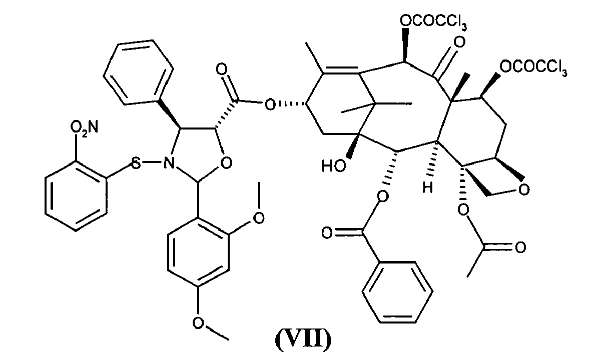
b) hydrolysis of the trichloroacetyl groups at the 7- and 10- positions of the compound of formula **(VII)** to give 2-(2,4-dimethoxyphenyl)-3-(2-nitrobenzensulfenyl)-4(*S*)-phenyl-5(*R*) -oxazolidine carboxylic acid, 10-deacetylbaccatin III 13-yl-ester **(VIII)** 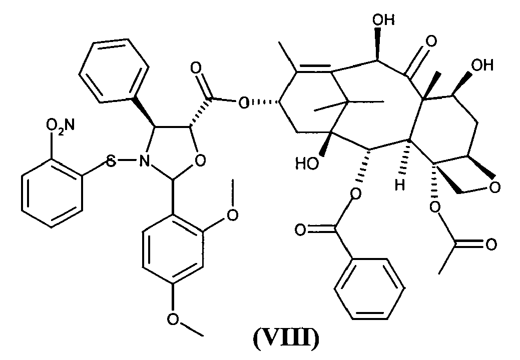
c) acid treatment of the compound formula **(VIII)** to give 10-deacetyl-N-debenzoyl-paclitaxel **(I).**

2. A process according to claim 1 wherein step a) is carried out in a solvent selected from an ether, an ester, an aromatic hydrocarbon or a halogenated aliphatic solvent.

3. A process according to claim 2 wherein the aliphatic halogenated hydrocarbon is methylene chloride.

4. A process according to any one of claims 1 - 3 wherein step a) is carried out in the presence of a condensing agent and an activating agent.

5. Process according to claim 4 wherein the condensing agent is dicyclohexylcarbodiiimide and the activating agent is 4-dimethylamino-pyridine.

6. A process according to any one of claims 1 to 5 wherein step b) is carried out with ammonium hydroxide in tetrahydrofuran as the solvent.

7. A process according to any one of claims from 1 to 6 wherein step c) is carried out with a methanol solution of aqueous hydrochloric acid.

8. 2-(2,4-Dimethoxyphenyl)-3-(2-nitrobenzensulfenyl)-4(*S*)-phenyl-5(*R*)-oxazolidine carboxylic acid, 10-deacetyl-7,10-bis-trichloroacetylbaccatin III 13-yl-ester **(VII)**

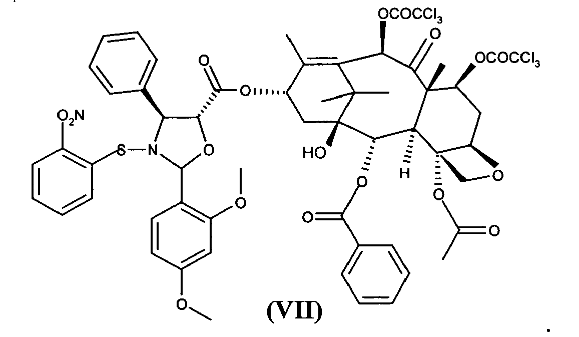

9. 2-(2,4-Dimethoxyphenyl)-3-(2-nitrobenzensulfenyl)-4(*S*)-phenyl-5(*R*)-oxazolidine carboxylic acid, 10-deacetylbaccatin III 13-yl-ester **(VIII)**
